# EUROPEAN PATENT APPLICATION

(11) **EP 2 889 381 A1**
(43) Date of publication of application: **01.07.2015**
(21) Application number: 13199634.0
(22) Date of filing: 27.12.2013
(51) Int. Cl.: C12Q 1/68

(54) **Detection method for species specific identification of nucleic acid of interest**

(71) Applicant: Université de Liège, 4031 Angleur (BE); Quality Partner S.A., 4040 Herstal (BE)
(72) Inventor: Daube, Georges, 4000 Liège (BE); Burteau, Sophie, 4000 Liège (BE); Delhalle, Laurent, 4040 Herstal (BE); Nezer, Carine, 4040 Herstal (BE)

(57) **Abstract**

A method for determining the species origin(s) of DNA in a sample comprising conducting metagenomic sequence analysis of the sample to identify DNA from at least one species, followed by species specific quantitative polymerase chain reaction (qPCR) to amplify and quantify a species specific target from the identified species DNA.

SEQUENCE LISTING

<110>  Université of Liege

<120>  Detection Methods

<130>  JA71063M-2013-55

<160>  44

<170>  PatentIn version 3.5

<210>  1
<211>  21
<212>  DNA
<213>  consensus

<400>  1
agtctatgtt tatatttacc t                                                21

<210>  2
<211>  21
<212>  DNA
<213>  consensus

<400>  2
aatctytgtt tatatttacc t                                                21

<210>  3
<211>  20
<212>  DNA
<213>  consensus

<400>  3
tcatcrtctt ccaargagcc                                                  20

<210>  4
<211>  51
<212>  DNA
<213>  consensus

<400>  4
cctatcccct gtgtgccttg gcagtctcag agtctatgtt tatatttacc t              51

<210>  5
<211>  51
<212>  DNA
<213>  consensus

<400>  5
cctatcccct gtgtgccttg gcagtctcag aatctytgtt tatatttacc t              51

<210>  6
<211>  59
<212>  DNA
<213>  consensus

<400>  6

ccatctcatc cctgcgtgtc tccgactcag agactgcact catcrtcttc caargagcc 59

<210> 7
<211> 59
<212> DNA
<213> consensus

<400> 7
ccatctcatc cctgcgtgtc tccgactcag atcgcaggct catcrtcttc caargagcc 59

<210> 8
<211> 60
<212> DNA
<213> consensus

<400> 8
ccatctcatc cctgcgtgtc tccgactcag acgctcgaca tcatcrtctt ccaargagcc 60

<210> 9
<211> 60
<212> DNA
<213> consensus

<400> 9
ccatctcatc cctgcgtgtc tccgactcag agacgcactc tcatcrtctt ccaargagcc 60

<210> 10
<211> 60
<212> DNA
<213> consensus

<400> 10
ccatctcatc cctgcgtgtc tccgactcag agcactgtag tcatcrtctt ccaargagcc 60

<210> 11
<211> 60
<212> DNA
<213> consensus

<400> 11
ccatctcatc cctgcgtgtc tccgactcag atcagacacg tcatcrtctt ccaargagcc 60

<210> 12
<211> 60
<212> DNA
<213> consensus

<400> 12
ccatctcatc cctgcgtgtc tccgactcag atatcgcgag tcatcrtctt ccaargagcc 60

<210> 13
<211> 60
<212> DNA
<213> consensus

<400> 13

ccatctcatc cctgcgtgtc tccgactcag cgtgtctcta tcatcrtctt ccaargagcc 60

<210> 14
<211> 27
<212> DNA
<213> consensus

<400> 14
gctagcagtc tatgtttata tttacct 27

<210> 15
<211> 27
<212> DNA
<213> consensus

<400> 15
actgcaaatc tytgtttata tttacct 27

<210> 16
<211> 20
<212> DNA
<213> consensus

<400> 16
tcatcrtctt ccaargagcc 20

<210> 17
<211> 18
<212> DNA
<213> Sus scrofa

<400> 17
ctgattgttg ctggtccc 18

<210> 18
<211> 18
<212> DNA
<213> Sus scrofa

<400> 18
gtttccgtcg tagcgtga 18

<210> 19
<211> 25
<212> DNA
<213> Sus scrofa

<400> 19
tgtaatgcat gtatgtggag acaaa 25

<210> 20
<211> 27
<212> DNA
<213> Gallus gallus

<400> 20

gctagcagtc tatgtttata tttacct                                          27

<210>  21
<211>  23
<212>  DNA
<213>  Gallus gallus

<400>  21
ttcatgcaga tcgcrgttga tcc                                              23

<210>  22
<211>  27
<212>  DNA
<213>  Bos taurus

<400>  22
actgcaaatc tctgtttata tttacct                                          27

<210>  23
<211>  29
<212>  DNA
<213>  Bos taurus

<400>  23
tgtttgtgga gggaaaacac tacatcctc                                        29

<210>  24
<211>  27
<212>  DNA
<213>  Ovis aries

<400>  24
actgcaaatc tttgtttata tttacct                                          27

<210>  25
<211>  30
<212>  DNA
<213>  Ovis aries

<400>  25
catgcttgtg gagacaaaac aataaatcct                                       30

<210>  26
<211>  29
<212>  DNA
<213>  Equus caballus

<400>  26
gcaaatctct gtttatattt acctgtttg                                        29

<210>  27
<211>  22
<212>  DNA
<213>  Equus caballus

<400>  27

ggtaatgatt gtttccgtcg tc                                                22

<210>  28
<211>  24
<212>  DNA
<213>  Equus caballus

<400>  28
ttcttgctgg tccagtggat ctaa                                              24

<210>  29
<211>  27
<212>  DNA
<213>  Meleagris gallopavo

<400>  29
gctagcagtc tatgtttata tttacct                                           27

<210>  30
<211>  22
<212>  DNA
<213>  Meleagris gallopavo

<400>  30
tgcagatttt agttcatccg gt                                                22

<210>  31
<211>  15
<212>  DNA
<213>  consensus

<400>  31
ggaactgatt gatca                                                        15

<210>  32
<211>  23
<212>  DNA
<213>  consensus

<400>  32
agtggaggag cyttgggyaa aag                                               23

<210>  33
<211>  1128
<212>  DNA
<213>  Coturnix chinensis

<400>  33
atgcaaaagc tagcagtcta tgtttatatt tacctgttcg tgcagatatc tgttgatccg      60

gtggctctcg atggcagtag tcagcccaca gagaacactg aaaaagacgg actgtgcaat     120

gcttgtacgt ggagacagaa cacaaaatcc tccagaatag aagccataaa aattcaaatc     180

ctcagcaaac tgcgcctgga acaagcacct aacattagca gggacgttat taaacaactt     240

ctacccaaag ctcctccact gcaggaactg attgatcagt acgacgtcca gagagatgac     300

agtagcgatg gctctttgga agatgatgac tatcatgcca caaccgaaac gattatcaca 360

atgcctacgg agtctgattt tcttgtacaa atggagggaa aaccaaaatg ttgcttcttt 420

aagtttagct ctaagataca atataacaaa gtagtaaagg cacaattatg gatatacttg 480

aggcaagtcc aaaaacctac aacggtgttt gtgcagatcc tgagactcat taaacccatg 540

aaagacggta caagatatac tggaattcga tctttgaaac ttgacatgaa cccaggcaat 600

ggtatctggc agagtattga tgtgaagaca gtcttgcaaa attggctcaa acagcctgaa 660

tccaacttag gcatcgaaat aaaagctttt gatgagaatg gacgagatct tgctgtaaca 720

ttcccaggac caggtgaaga cggatcgaac ccatttttag aggtcagagt tacagataca 780

ccaaaacggt cccgcagaga ttttggcctt gactgtgatg agcactcaac agaatctcga 840

tgttgtcgct accccttgac ggtggatttt gaagcttttg gatgggactg gattatagcg 900

cctaaaagat acaaagcaaa ttactgctct ggagaatgcg aatttgtatt tctacagaaa 960

taccctcaca ctcacctggt gcaccaagca aatccaagag gttcagcagg cccttgctgc 1020

acacccacca agatgtcccc tataaatatg ctgtatttca atggaaaaga acaaataata 1080

tatggaaaga taccagctat ggttgtagat cgttgcgggt gctcatga 1128

<210> 34
<211> 1128
<212> DNA
<213> Gallus gallus

<400> 34
atgcaaaagc tagcagtcta tgtttatatt tacctgttca tgcagatcgc ggttgatcca 60

gtggctctgg atggcagtag tcagcccaca gagaacgctg aaaaagacgg actgtgcaat 120

gcttgtacgt ggagacagaa tacaaaatcc tccagaatag aagccataaa aattcaaatc 180

ctcagcaaac tgcgcctgga acaagcacct aacattagca gggacgttat taagcagctt 240

ttacccaaag ctcctccact gcaggaactg attgatcagt atgatgtcca gagggacgac 300

agtagcgatg gctctttgga agacgatgac tatcatgcca caaccgagac gattatcaca 360

atgcctacgg agtctgattt tcttgtacaa atggagggaa aaccaaaatg ttgcttcttt 420

aagtttagct ctaaaataca atataacaaa gtagtaaagg cacaattatg gatatacttg 480

aggcaagtcc aaaaacctac aacggtgttt gtgcagatcc tgagactcat taagcccatg 540

aaagacggta caagatatac tggaattcga tctttgaaac ttgacatgaa cccaggcact 600

ggtatctggc agagtattga tgtgaagaca gtgctgcaaa attggctcaa acagcctgaa 660

tccaatttag gcatcgaaat aaaagctttt gatgagactg gacgagatct tgctgtcaca 720

ttcccaggac cgggtgaaga tggattgaac ccatttttag aggtcagagt tacagacaca 780

ccgaaacggt cccgcagaga ttttggcctt gactgtgatg agcactcaac ggaatcccga 840

tgttgtcgct acccgctgac agtggatttc gaagcttttg gatgggactg gattatagca 900

cctaaaagat acaaagccaa ttactgctcc ggagaatgtg aatttgtgtt tctacagaaa 960

tacccgcaca ctcacctggt acaccaagca aatcccagag gctcagcagg cccttgctgc 1020

acacccacca agatgtcccc tataaacatg ctgtatttca atggaaaaga acaaataata 1080

tatggaaaga taccagccat ggttgtagat cgttgcgggt gctcatga 1128

<210> 35
<211> 1128
<212> DNA
<213> Anas platyrhynchos

<400> 35
atgcaaaagc tagcagtcta tgtttatatt tacctgttca tgctgatttc agttgatccg 60

gtggctcttg atgacggcag tcagcccaca gagaacgctg aaaaagatgg actgtgcaat 120

gcttgtacgt ggagacagaa tacaaaatct tccagaatag aagccataaa aattcaaatc 180

ctcagcaaac tgcgcctgga acaagctcct aacattagca gggatgttat taagcaactt 240

ttacccaaag ctcctccact acaggaactg attgatcaat atgacgtcca gagagacgac 300

agtagcgatg gctctttgga agacgatgac tatcatgcca caactgaaac gattatcaca 360

atgcctacag agtctgattt tcttgtacaa atggagggaa aaccaaaatg ttgcttcttt 420

aagtttagct ctaaaataca atataacaaa gtagtaaagg cacaattgtg gatatacttg 480

aggcaagtcc aaaaacctac aacagtgttt gtgcagatcc tgagacttat taagcccatg 540

aaagacggtc caagatatac tggaattcga tctttgaaac ttgacatgaa cccaggcact 600

ggtatttggc agagtattga tgtgaagaca gtgttgcaaa attggctcaa acagcctgaa 660

tccaatttag gcatcgaaat aaaagctttt gatgagaatg gacgagatct tgctgtaact 720

ttcccaggac caggtgaaga tggattgaat ccatttttag aggtcagagt tacagacaca 780

ccgaaacgat cccgcagaga ttttggcctt gactgcgatg agcactcgac agaatcccga 840

tgttgtcgct acccactgac cgtggatttt gaagcttttg gatgggactg gattatcgcc 900

cctaaaagat acaaagccaa ttactgctct gaagaatgcg aatttgtatt tctacagaaa 960

tacccgcaca ctcatcttgt gcaccaagcg aatcctagag gatcggcagg cccctgctgc 1020

acgcccacca agatgtcccc cataaatatg ttgtatttca atggaaaaga acaaataata 1080

tacggaaaga taccagccat ggttgtagat cgttgcgggt gctcatga 1128

<210> 36
<211> 1128
<212> DNA
<213> Anser anser

<400> 36

atgcaaaagc tagcagtcta tgtttatatt tacctgttca tgctgatttc agttgatccg 60

gtggctcttg atgacggtag tcagcccaca gagaatgctg aaaaagatgg actgtgcaat 120

gcttgtacat ggagacagaa tacaaaatct tccagaatag aagccataaa aattcaaatc 180

ctcagcaaac tgcgtctgga acaagctcct aacattagca gggatgttat taaacaactt 240

ttacccaaag ctcctccact acaggaactg attgatcaat atgacgtcca gagagatgac 300

agtagcgatg gctctttgga agacgatgac tatcatgcca caactgaaac gattatcaca 360

atgcctacag agtctgattt tcttgtacaa atggagggaa aaccaaaatg ttgcttcttt 420

aagtttagct ctaaaataca atataacaaa gtagtaaagg cacaattgtg gatttacttg 480

aggcaagtcc aaaaacctac aacagtgttt gtgcagatcc tgagacttat taagcccatg 540

aaagacggga caagatatac tggaattcgg tctttgaaac ttgacattga cccaggcgct 600

ggttttgggc agagtattga tgtgaagaca gtgttgcaaa attggctcaa acagcctgaa 660

tccaatttag gcatcgaaat aaaagctttt gatgagaatg gacgagatct tgctgtaact 720

ttcccaggac caggtgaaga tggattgaat ccatttttag aggtcagagt tacagacaca 780

ccgaaacgat cccgcagaga ttttggcctt gactgcgatg agcactcgac agaatccaga 840

tgttgtcgct acccactaac tgtggatttt gaagcttttg gatgggactg gattatcgca 900

cctaaaagat acaaagccaa ttactgcttt ggagaatgtg aatttgtatt tctacagaaa 960

tatccgcaca ctcatcttgt acaccaagca aatcctagag gctcggcagg cccctgctgc 1020

acgcccacca agatgtcccc cataaatatg ttgtatttca atggaaaaga acaaataata 1080

tacggaaaga taccagccat ggttgtagat cgttgcgggt gctcatga 1128

<210> 37
<211> 1140
<212> DNA
<213> Cervus elaphus

<400> 37
gattttaaaa ccatgcaaaa actgcaaatc tgtgtttata tttacctatt tatgctgatt 60

gttgctggcc cagtggatct gaatgagaac agcgagcaga aggaaaatgt ggaaaaagag 120

gggctgtgta atgcatgttt gtggagacaa aacactaaat ccttaaggct agaagccata 180

aaaatccaaa tcctcagtaa acttcgcctg gaaacagctc ctaacatcag caaagatgct 240

ataagacaac ttctgcccaa agctcctcca ctccgggaac tgattgatca gtacgatgtc 300

cagagagatg acagcagtga cggctccttg gaagatgatg actaccacgc tacgacggaa 360

acggtcatta ccatgcccac ggagtctgat cttctaacgc aagtggaagg aaaacccaaa 420

tgttgcttct ttcagtttag ctctaagata caatacaata aagtcgtaaa ggcccaactg 480

tggatatatc tgagacctgt caagactcct acgacggtgt ttgtgcaaat cctgagactc 540

atcaaaccca tgaaagacgg tacaaggtat actggaatcc gatctctgaa acttgacatg 600

aacccaggca ctggtatttg gcagagcatt gacgtgaaga cagtgttgca aaactggctc 660

aaacaacctg aatccaactt aggcattgag atcaaagctt tagatgagaa tggccatgat 720

cttgctgtaa ccttcccaga accaggagaa gatggactga atcctttttt agaagtcaag 780

gtaacagaca caccaaaaag atctaggaga gattttgggc tcgattgtga tgagcactcc 840

acagaatctc gatgctgtcg ttaccctcta actgtggatt ttgaagctct tggatgggat 900

tggattattg cacctaaaag atataaggcc aattactgct ctggagaatg tgaatttgtg 960

tttttgcaaa agtatcctca tacccatctt gtgcaccaag cgaaccccag tggttcagcc 1020

ggcccctgct gtactcctac aaagatgtct ccaattaata tgctatattt taatgacaaa 1080

gaacaaataa tacatgggaa gattccagct atggtagtag atcgctgtgg gtgctcatga 1140

<210> 38
<211> 1128
<212> DNA
<213> Antilocapra americana

<400> 38
atgcaaaaac tgcaaatcta tgtttatatt tacctatcta tgctgattgt tgctggcccc 60

gtggatctga atgagaacag cgagcagaag gaaaatgggg aaaaagaggg gctgtgtaat 120

gcatgtttgt ggagacaaaa cactaaatcc tcaagactag aagccataaa aatccaaatc 180

ctcagtaaac tgcgcctgga aacagctcct aacatcagca aagatgctat aagacaactt 240

ttgcccaaag ctcccccgct ccgggaactg atcgatcagt acgacgtcca gagagatgac 300

agcagtgacg gctccttgga agacgatgac taccacgcta cgacggaaac ggtcattacc 360

atgcccacgg agtctgatct tctaactcaa gtggaaggaa aacccaaatg ttgcttcttt 420

caatttagct ctaagataca atacaataaa gtagtaaagg cccagctgtg gatatatctg 480

agacctgtca agactcctac gacagtgttt gtacaaatcc tgagactcat caaacccatg 540

aaagacggta caaggtatac tggaatccga tctctgaagc ttgacatgaa cccaggcgct 600

ggtatttggc agagcattga tgtgaagaca gtgttgcaaa actggctcaa acaacctgaa 660

tccaacttag gcattgaaat caaagcttta gatgagaatg gccatgatct tgctgtaacc 720

ttcccagaac caggagaaga tggactgaac ccttttttag aagtcaaggt agcagacaca 780

ccaaaaagat ctaggagaga ttttgggctc gattgtgatg agcactccac agaatctcga 840

tgctgtcgtt accctctaac tgtggatttt gaagcttttg gatgggattg gatcattgca 900

cctaaaagat ataaggccaa ttactgctct ggagaatgtg aatttgtgtt tttacaaaag 960

tatcctcata cccatcttgt gcaccaagca aaccccagag gttcagcagg cccctgctgt 1020

actcctacaa agatgtctcc aattaatatg ctatatttta atggcaaaga acaaataata 1080

tacgggaaga ttccagccat ggtagtagat cgctgtgggt gctcatga 1128

<210> 39
<211> 1140
<212> DNA
<213> Bos taurus

<400> 39
gattttaaaa ccatgcaaaa actgcaaatc tctgtttata tttacctatt tatgctgatt 60

gttgctggcc cagtggatct gaatgagaac agcgagcaga aggaaaatgt ggaaaaagag 120

gggctgtgta atgcatgttt gtggagggaa aacactacat cctcaagact agaagccata 180

aaaatccaaa tcctcagtaa acttcgcctg gaaacagctc ctaacatcag caaagatgct 240

atcagacaac ttttgcccaa ggctcctcca ctcctggaac tgattgatca gttcgatgtc 300

cagagagatg ccagcagtga cggctccttg gaagacgatg actaccacgc caggacggaa 360

acggtcatta ccatgcccac ggagtctgat cttctaacgc aagtggaagg aaaacccaaa 420

tgttgcttct ttaaatttag ctctaagata caatacaata aactagtaaa ggcccaactg 480

tggatatatc tgaggcctgt caagactcct gcgacagtgt ttgtgcaaat cctgagactc 540

atcaaaccca tgaaagacgg tacaaggtat actggaatcc gatctctgaa acttgacatg 600

aacccaggca ctggtatttg gcagagcatt gatgtgaaga cagtgttgca gaactggctc 660

aaacaacctg aatccaactt aggcattgaa atcaaagctt tagatgagaa tggccatgat 720

cttgctgtaa ccttcccaga accaggagaa gatggactga ctcctttttt agaagtcaag 780

gtaacagaca caccaaaaag atctaggaga gattttgggc ttgattgtga tgaacactcc 840

acagaatctc gatgctgtcg ttaccctcta actgtggatt ttgaagcttt tggatgggat 900

tggattattg cacctaaaag atataaggcc aattactgct ctggagaatg tgaatttgta 960

tttttgcaaa agtatcctca tacccatctt gtgcaccaag caaaccccag aggttcagcc 1020

ggcccctgct gtactcctac aaagatgtct ccaattaata tgctatattt taatggcgaa 1080

ggacaaataa tatacgggaa gattccagcc atggtagtag atcgctgtgg gtgttcatga 1140

<210> 40
<211> 1128
<212> DNA
<213> Ovis aries

<400> 40
   atgcaaaaac tgcaaatctt tgtttatatt tacctattta tgctgcttgt tgctggccca 60
   gtggatctga atgagaacag cgagcagaag gaaaatgtgg aaaaaaaggg gctgtgtaat 120
   gcatgcttgt ggagacaaaa caataaatcc tcaagactag aagccataaa aatccaaatc 180
   ctcagtaagc ttcgcctgga aacagctcct aacatcagca aagatgctat aagacaactt 240
   ttgcccaagg ctcctccact ccgggaactg attgatcagt acgatgtcca gagagatgac 300
   agcagcgacg gctccttgga agacgatgac taccacgtta cgacggaaac ggtcattacc 360
   atgcccacgg agtctgatct tctagcagaa gtgcaagaaa aacccaaatg ttgcttcttt 420
   aaatttagct ctaagataca acacaataaa gtagtaaagg cccaactgtg gatatatctg 480
   agacctgtca agactcctac aacagtgttt gtgcaaatcc tgagactcat caaacccatg 540
   aaagacggta caaggtatac tggaatccga tctctgaaac ttgacatgaa cccaggcact 600
   ggtatttggc agagcattga tgtgaagaca gtgttgcaaa actggctcaa acaacctgaa 660
   tccaacttag gcattgaaat caaagcttta gatgagaatg gtcatgatct tgctgtaacc 720
   ttcccagaac caggagaaga aggactgaat ccttttttag aagtcaaggt aacagacaca 780
   ccaaaaagat ctaggagaga ttttgggctt gattgtgatg agcactccac agaatctcga 840
   tgctgtcgtt accctctaac tgtggatttt gaagcttttg gatgggattg gattattgca 900
   cctaaaagat ataaggccaa ttactgctct ggagaatgtg aattttattt tttgcaaaag 960
   tatcctcata cccatcttgt gcaccaagca aaccccaaag gttcagccgg cccttgctgt 1020
   actcctacaa agatgtctcc aattaatatg ctatatttta atggcaaaga acaaataata 1080
   tatgggaaga ttccaggcat ggtagtagat cgctgtgggt gctcatga 1128

<210> 41
<211> 1255
<212> DNA
<213> Capra hircus

<400> 41
ctggtgtggc aagttgtctc tcagactggg caggcattaa cgtttggctt ggcgttactc 60

aaaagcaaaa gaaaagtaaa aggaagaagt aagagcaagg aaaaagattg tattgatttt 120

aaaaccatgc aaaaactgca aatctttgtt tatatttacc tatttatgct gcttgttgct 180

ggcccagtgg atctgaatga gaacagcgag cagaaggaaa atgtggaaaa aaaggggctg 240

tgtaatgcat gcttgtggag acaaaacaat aaatcctcaa gactagaagc cataaaaatc 300

caaatcctca gtaagcttcg cctggaaaca gctcctaaca tcagcaaaga tgctataaga 360

caacttttgc ccaaggctcc tccactccgg gaactgattg atcagtacga tgtccagaga 420

gatgacagca gcgacggctc cttggaagac gatgactacc acgttacgac ggaaacggtc 480

attaccatgc ccacggagtc tgatcttcta gcagaagtgc aagaaaaacc caaatgttgc 540

ttctttaaat ttagctctaa gatacaacac aataaagtag taaaggccca actgtggata 600

tatctgagac ctgtcaagac tcctacaaca gtgtttgtgc aaatcctgag actcatcaaa 660

cccatgaaag acggtacaag gtatactgga atccgatctc tgaaacttga catgaaccca 720

ggcactggta tttggcagag cattgatgtg aagacagtgt tgcaaaactg gctcaaacaa 780

cctgaatcca acttaggcat tgaaatcaaa gctttagatg agaatggtca tgatcttgct 840

gtaaccttcc cagaaccagg agaagaagga ctgaatcctt ttttagaagt caaggtaaca 900

gacacaccaa aaagatctag gagagatttt gggcttgatt gtgatgagca ctccacagaa 960

tctcgatgct gtcgttaccc tctaactgtg gattttgaag cctttggatg ggattggatt 1020

attgcaccta aaagatataa ggccaattac tgctctggag aatgtgaatt tttatttttg 1080

caaaagtatc ctcataccca tcttgtgcac caagcaaacc ccaaaggttc agccggccct 1140

tgctgtactc ctacaaagat gtctccaatt aatatgctat attttaatgg caaagaacaa 1200

ataatatatg ggaagattcc aggcatggta gtagatcgct gtgggtgctc atgag 1255

<210> 42
<211> 1128
<212> DNA
<213> Felis catus

<400> 42
atgcagaaac tgcaaatcta tgtttatatt tacctgttta tgctgattgt tgctggtcca 60

gtggatctaa atgagaacag tgagcaaaaa gaaaatgtgg aaaaagaggg gctgtgcaat 120

gcatgtactt ggagacaaaa cactaaatct tcaagaatag aagccataaa aattcagatc 180

ctcagtaaac ttcgcctgga aacggctccc aacatcagca aagatgctat aagacaactt 240

ttgcccaaag ctcctccgct ccgggaactg attgatcagt acgatgtcca gagagatgac 300

agcagtgatg gctctttgga agacgacgat taccacgcta ccacggaaac gatcattacc 360

atgcccaccg agtctgatct tctaatgcaa gtggaaggaa aacccaaatg ttgcttcttt 420

aaatttagct ctaaaataca atataataaa gtcgtaaagg cccaactgtg gatatatctg 480

agacccgtca agactcctac aacagtgttt gtgcaaatcc tgagactcat caaacccatg 540

aaagacggta caaggtatac tggaatccga tctctgaaac ttgacatgaa cccaggcact 600

ggtatttggc agagcattga tgtgaagaca gtgttgcaaa attggctcaa acagcctgaa 660

tccaacttag gcattgaaat caaagcttta gatgagaatg gtcatgatct tgctgtaacc 720

ttcccaggac caggagaaga tgggctgaat ccctttttag aagtcaaggt gacagacaca 780

ccaaaaagat ccagacgaga ttttgggctt gactgtgatg agcactcaac agaatctcgg 840

tgctgtcgtt accctctcac tgtggatttt gaagcttttg gatgggattg gattattgca 900

cccaaaagat ataaggccaa ttactgctct ggagagtgtg aatttgtgtt tttacaaaaa 960

tatcctcata ctcatcttgt acaccaagca aaccccagag gttcagcagg cccctgctgt 1020

actcccacca agatgtctcc aattaatatg ctatatttta atggcaagga acaaataata 1080

tatgggaaaa ttccagccat ggtagtagat cgctgtgggt gctcatga 1128

<210> 43
<211> 1128

<212>  DNA
<213>  Equus caballus

<400>  43
atgcaaaaac tgcaaatctc tgtttatatt tacctgtttg tgctgattct tgctggtcca      60

gtggatctaa atgagaacag cgagcaaaaa gaaaatgtgg aaaaagaggg gctgtgcaat     120

gcatgtactt ggagacaaaa cactaaatct tcaagaatag aagccataaa aattcagatc     180

ctcagtaaac tgcgcctgga aacagctcct aacatcagca aagatgctat tagacaactt     240

ttgcccaaag ctcctccact ccgggaactg attgatcagt acgatgtcca gagagatgac     300

agcagtgatg gctctttgga agatgatgat taccacgcga cgacggaaac aatcattacc     360

atgcctacag agtctgatct tctaatgcaa gtggaaggaa aacccaaatg ttgcttcttt     420

aaatttagct ctaaaataca atacaataaa gtagtaaagg cccaactgtg gatatatctg     480

agacccgtca agactcctac aacagtgttt gtgcaaatcc tgagactcat caaacccatg     540

aaagacggta caaggtatac tggaatccga tctctgaaac ttgacatgaa cccaggcgct     600

ggtatttggc agagcattga tgtgaagaca gtgttgcaaa attggctcaa acagcctgaa     660

tccaacttag gcattgaaat caaagcttta gatgagaatg gtcatgatct tgctgtaacc     720

ttcccaagac caggagaaga tgggctgaat ccatttttag aagttaaggt aacagacaca     780

ccaaaacgat ccagaagaga ttttggactt gactgtgatg agcactccac agaatctcga     840

tgctgtcgtt accctctaac tgtggatttt gaagcttttg gatgggattg gattattgca     900

cccaaaagat ataaggccaa ttactgctct ggagagtgtg aatttgtatt tttacaaaaa     960

tatcctcaca ctcatcttgt acaccaagca aaccccagag gttcagcagg cccctgctgt    1020

actcccacaa agatgtctcc aattaatatg ctatatttta atggcaaaga acaaataata    1080

tatgggaaaa ttccagccat ggtagtagat cgctgtgggt gctcatga                 1128


<210>  44
<211>  1128
<212>  DNA
<213>  Sus scrofa

<400>  44
atgcaaaaac tgcaaatcta tgtttatatt tacctgttta tgctgattgt tgctggtccc      60

gtggatctga atgagaacag cgagcaaaag gaaaatgtgg aaaaagaggg gctgtgtaat     120

gcatgtatgt ggagacaaaa cactaaatct tcaagactag aagccataaa aattcaaatc     180

ctcagtaaac ttcgcctgga aacagctcct aacattagca aagatgctat aagacaactt     240

ttgcccaaag ctcctccact ccgggaactg attgatcagt acgatgtcca gagagatgac     300

agcagtgatg gctccttgga agatgatgat tatcacgcta cgacggaaac gatcattacc     360

atgcctacag agtctgatct tctaatgcaa gtggaaggaa aacccaaatg ctgcttcttt     420

aaatttagct ctaaaataca atacaataaa gtagtaaagg cccaactgtg gatatatctg 480

agacccgtca agactcctac aacagtgttt gtgcaaatcc tgagactcat caaacccatg 540

aaagacggta caaggtatac tggaatccga tctctgaaac ttgacatgaa cccaggcact 600

ggtatttggc agagcattga tgtgaagaca gtgttgcaaa attggctcaa acaacctgaa 660

tccaacttag gcattgaaat caaagcttta gatgagaatg gtcatgatct tgctgtaacc 720

ttcccaggac caggagaaga tgggctgaat ccctttttag aagtcaaggt aacagacaca 780

ccaaaaagat ccaggagaga ttttggactc gactgtgatg agcactcaac agaatctcga 840

tgctgtcgtt accctctaac tgtggatttt gaagcttttg gatgggactg gattattgca 900

cccaaaagat ataaggccag ttactgctct ggagagtgtg aatttgtatt tttacaaaaa 960

taccctcaca ctcatcttgt gcaccaagca aaccccagag gttcagcagg cccctgctgt 1020

actcccacaa agatgtctcc aatcaatatg ctatatttta atggcaaaga acaaataata 1080

tatgggaaaa ttccagccat ggtagtagat cgctgtgggt gctcatga 1128

## Description

The invention relates to analytical methods that have been developed to identify and quantify the more frequent animal species present in ground meat, meat preparations and meat products and to quantify the proportion of them.

Species identification is a major concern due to the increased awareness of consumers regarding the composition of foods, and the need to verify labelling statements. Ground meat, meat preparations and processed meat products are susceptible targets for fraudulent labelling due to the economic profit that results from selling cheaper meats as partial or total replacement for high-valued meats. Several analytical methods are used for authentication of meat and meat products. The analytical methods include polymerase chain reaction, chromatography, mass spectrometry, microscopy, spectroscopy, electronic spin resonance, and enzymatic assays. In species determination, analysis of DNA and protein are common practices.

Among DNA-based methods, Polymerase Chain Reaction (PCR) is an effective technique that is highly accurate and relatively fast. Conventional qualitative PCR methods have a satisfactory performance in the qualitative detection of meat species. However, the need for methods that give quantitative results has arisen following the introduction of labelling obligations. Moreover, quantification is also necessary for the detection of intentional adulteration of meat products or accidental contamination in the production line. There is therefore a need for improved methods for the quantitative determination of the species origin(s) of meat and meat products.

The invention provides a method for determining the species origin(s) of DNA in a sample comprising conducting metagenomic sequence analysis of the sample to identify DNA from the present species and the relative proportions of them, followed by species specific quantitative polymerase chain reaction (qPCR) to amplify and quantify a species specific target from the identified species DNA.

Among new generation molecular technologies the metagenomic analysis has emerged as a powerful tool. Metagenomics applies a suite of genomic technologies and bioinformatics tools to directly access the genetic content of entire communities of organisms. This technology is commonly used to describe microbial community profiles of various ecosystems like seas, soils, rumen, faeces, but also to study complex microbiota of foodstuff. The invention applies the technique of metagenomics in the first stage of a sequential identification, and quantitation of the origin of DNA in a sample.

In some embodiments, the metagenomic analysis comprises amplification and sequencing of a metagenomic target DNA sequence, said metagenomic target DNA sequence being selected to allow for: i) co-amplification of the metagenomic target DNA sequence from a plurality of species using a set of conserved, common primers; and ii) the identification of species origin of any amplified metagenomic target DNA on the basis of its sequence. Preferably the metagenomic target DNA sequence is sequenced by a process for massively parallel sequencing. In some embodiments, the process for massively parallel sequencing is selected from emulsion sequencing, and solid phase sequencing.

In the optional second stage of the method of the invention, real-time, or quantitative PCR (qPCR) is used to quantitate the amount of DNA of the species identified in the first stage as being present in the sample. Real-time PCR has demonstrated the highest improvement among PCR-based quantitative methods in recent years. In real-time PCR, the exponential amplification of target-specific DNA is monitored by an increased fluorescence signal. If the species under study is present in the matrix, a specific signal can be observed. As well as meeting the need for quantitative determination in meat species, this technique also has other advantages like higher sensitivity and specificity.

Quantitative PCR may be based on the use of intercalating dyes, or the use of a labelled reporter primer and/or probe. In some embodiments of the invention, therefore, the qPCR step is selected from qPCR using an intercalating dye and qPCR using a labelled reporter primer or probe. In embodiments based on intercalating fluorescent dyes, the dye may be SYBRGreen or EvaGreen. Such methods require the greatest sequence specificity due to the fact that these types of dyes bind to all double-stranded DNA present, including any nonspecific PCR products and the primer-dimer complex.

In other, preferable embodiments, the real-time quantitative PCR procedure for species identification is generally based on the use of a labelled reporter primer or probe. Preferably, the labelled reporter primer or probe is part of a fluorescence/quencher reporter system or other fluorescence reporter system in which amplification of the species specific DNA target results in a reduction or elimination of fluorescence quenching, or other increase in fluorescence, to result in a detectable fluorescent signal. For example a TaqMan fluorogenic probe (also referred to herein as a hydrolysis probe (the 5' to 3' exonuclease activity of the polymerase degrades the probe that has annealed to the template. Degradation of the probe releases the fluorophore from it and breaks the close proximity to the quencher, thus relieving the quenching effect and allowing fluorescence of the fluorophore)) may be used as the reporter system. This method uses an additional oligonucleotide, which is also bound specifically to the target DNA sequence during the annealing step. This oligonucleotide has both a reporter fluorescent dye and a quencher dye attached. The accumulation of specific PCR products is detected by hybridization and cleavage of a double-labelled fluorogenic probe during the amplification reaction.

Other alternative reporter systems may be employed. For example, the reporter system may comprise a molecular beacon probe, a pair of dual hybridization probes, an amplifluor primer system, a scorpion primer system, a light-upon-extension system or a QZyme primer system. Other suitable systems will be know to the skilled person and may be used to provide quantitative real-time PCR reporting.

Any suitable target sequences may be used for either or both stages of the process of the invention. To be suitable for stage 1 of the process (metagenomic analysis) the target must allow for co-amplification from a plurality of species using a set of conserved, common primers. Preferably a single pair of primers (one forward primer and one reverse primer) is used to co-amplify the metagenomic target DNA sequence from substantially all species of interest. However, the invention is not limited to the use of just a single pair of primers. Metagenomic analysis may be improved, and expanded in scope by the addition of multiple forward and/or reverse primers which can allow for a broader range of species specific sequences that can be amplified. Multiple primers can also be used to ensure that amplification efficiency is broadly similar for all species of interest in order to eliminate or reduce over-representation of one species, possibly at the expense of another, in the amplification process.

The metagenomic target sequence is preferably a sequence which has the same number of copies per genome in each of the species suspected as being present in the sample, so as to allow for a determination of the relative proportions of the different identified DNAs by the metagenomic analysis.

The skilled person is able to determine appropriate primer pairs for the metagenomic analysis, based upon an examination of a line-up of sequences from a homologous gene shared by the species of interest. Highly conserved regions of the gene of interest can be examined for the possibility of providing a relatively small number of primers capable of amplifying target from a large number of species. Preferably a small number of primers, for example 2 or 3 primers may be adequate to amplify a plurality of target sequences from e.g. at least 2, 3, 4, 5, 6, 7, 8, 9, 10, 11, 12 or 13 or more species.

Primers may include one or more degenerate nucleotide bases in order to increase the range of species whose target DNA may be amplified in the metagenomic analysis. Primers may also be modified by the addition of adaptors or tags, or universal sequences for use in manipulation of amplicon products, including sequencing of the products.

In the metagenomic analysis, following amplification of target sequences, the amplicons so produced are sequenced to determine species origin. A further requirement, therefore, of the metagenomic target DNA sequence is that it allows for the identification of species origin of any amplified metagenomic target DNA on the basis of its sequence. This may be achieved by providing PCR primers from highly conserved regions (as discussed above), which flank regions of lower conservation amongst the various homologues of the species of interest, such that the specific sequence of the amplified target, between the primer sequences, may be used to identify the species of origin.

The inventors have found that the myostatin (MSTN) gene is a suitable gene for use in the process of the invention, particularly in the identification and quantitation of species in meat products. The MSTN gene encodes the myostatin protein (also known as growth differentiation factor 8 or GDF-8). Myostatin is a secreted growth differentiation factor that is a member of the TGF beta protein family that inhibits muscle differentiation and growth in the process known as myogenesis. The gene is classified as a housekeeping gene, that is a group of genes coding mainly for essential proteins in the cellular basic function. These genes include conserved area useful for conserved primers design and variable area required for species identification and present in single copy in the genome, an important feature for the quantification method.

In some embodiments the myostatin gene is used in the invention to identify and quantitate DNA from at least one species from a genus selected from *Sus, Gallus, Bos, Ovis, Equus, Meleagris, Felis, Capra, Antilocapra, Cervus, Anas, Anser* and *Coturnix,* for example, *Sus scrofa, Gallus gallus, Bos Taurus, Ovis aries, Equus caballus, Meleagris gallopavo, Felis catus, Capra hircus, Antilocapra Americana, Cervus elaphus, Anas platyrhyncho, Anser anser* and *Coturnix chinensi.*

In some embodiments, the metagenomic target DNA sequence is therefore from the MSTN gene. Preferably, the metagenomic target DNA sequence is amplified by a set of conserved primers comprising at least a primer comprising the sequence of SEQ ID NO. 1 or SEQ ID NO. 2, and a primer comprising the sequence of SEQ ID NO. 3. In some embodiments the metagenomic target DNA sequence is amplified by a set of conserved primers comprising a primer comprising the sequence of SEQ ID NO. 1 and a primer comprising the sequence of SEQ ID NO. 2 and a primer comprising the sequence of SEQ ID NO. 3. In some embodiments, the primers consist of the sequences of SEQ ID NO. 1, SEQ ID NO. 2 and SEQ ID NO. 3. In some embodiments the primers having or comprising the sequences of SEQ ID No's 1, 2 or 3 are further modified by the addition of adaptors, tags or universal sequences.

In some embodiments the set of primers for amplification of the metagenomic target DNA comprises a set of forward primers having the sequences of SEQ ID NO.s 4 and 5, and a set of reverse primers having the sequences of SEQ ID NO.s 6 to 13.

The MSTN gene is also suitable for use in the second stage of the process of the invention, that is the qPCR stage. Accordingly, in some embodiments, the species specific target DNA sequence is amplified by a set of primers and the resulting amplicon is detected by a labelled hybridisation probe, the primers and probes being selected from the following:
a) primers comprising or consisting of the sequences of SEQ ID NOS. 17 and 18 and a probe comprising or consisting of the sequence of SEQ ID NO. 19 for the quantitation of DNA sequences from pork (*Sus scrofa*); or
b) primers comprising or consisting of the sequences of SEQ ID NOS. 20 and 16 and a probe comprising or consisting of the sequence of SEQ ID NO. 21 for the quantitation of DNA sequences from chicken (*Gallus gallus*); or
c) primers comprising or consisting of the sequences of SEQ ID NOS. 22 and 16 and a probe comprising or consisting of the sequence of SEQ ID NO. 23 for the quantitation of DNA sequences from beef (*Bos taur us*); or
d) primers comprising or consisting of the sequences of SEQ ID NOS. 24 and 16 and a probe comprising or consisting of the sequence of SEQ ID NO. 25 for the quantitation of DNA sequences from sheep (*Ovis aries*); or
e) primers comprising or consisting of the sequences of SEQ ID NOS. 26 and 27 and a probe comprising or consisting of the sequence of SEQ ID NO. 28 for the quantitation of DNA sequences from horse (*Equus caballus*); or
f) primers comprising or consisting of the sequences of SEQ ID NOS. 29 and 16 and a probe comprising or consisting of the sequence of SEQ ID NO. 30 for the quantitation of DNA sequences from turkey (*Meleagris gallopavo*); or
g) primers comprising or consisting of the sequences of SEQ ID NOs. 14 and/or 15 and 16 and a probe comprising or consisting of the sequence of SEQ ID NO. 31 or 32 for the quantitation of DNA from all species in the sample.

Preferably, the primers and probes consist of the sequences of SEQ ID NOs. 14 to 32 respectively.

In some embodiments, the forward primers selected for the various species may include primers with some degeneracy and/or may include a mixture of primers. Conveniently, for the quantitation of DNA sequences from chicken, beef, sheep or turkey, the forward primer noted above may be replaced by a mixture of primers having the sequences of SEQ ID NO.s 14 and 15.

In preferred embodiments, the quantitation of species specific target DNA sequences is compared to a control or reference target sequence. Preferably the control or reference target sequence is part of the same gene as the species specific target sequence. In some embodiments, therefore, a control or reference target sequence is part of the MSTN gene sequence. In some embodiments the control or reference target sequence is co-amplified with the species specific target sequence, and is detected in real time (qPCR). In some embodiments a control or reference target sequence is amplified using a set of primers comprising or consisting of the sequences of SEQ ID NOs 14, 15 and 16, and detected using a probe comprising or consisting of the sequence of SEQ ID NO. 31 or SEQ ID NO. 32.

In another aspect, the invention provides the use of the primers as discussed above (comprising or consisting of the sequence of SEQ ID NOs 1, 2 and 3, or SEQ ID NOs 4 to 13) with the primers and probes also discussed herein (comprising or consisting of the sequences of SEQ ID NOs 14 to 32) for the sequential metagenomic identification and relative proportion determination and species specific quantitation of DNA in a sample, the selection of species specific primers and probes employed for quantitation being chosen on the basis of the metagenomic identification of species DNA present in the sample. Preferably the sample is a food sample such as a meat sample.

In a further aspect, the invention provides a kit for metagenomic identification of DNA present in a sample, comprising a set of primers for the metagenomic amplification of a metagenomic target sequence in the myostatin gene, said metagenomic target sequence being selected to allow for: i) co-amplification of the metagenomic target DNA sequence from a plurality of species using a set of conserved, common primers; ii) the identification of species origin of any amplified metagenomic target DNA on the basis of its sequence; and optionally iii) the relative proportions between the different identified DNA. In some embodiments the metagenomic target sequence is selected to allow for amplification of DNA from at least 2, 3, 4, 5, 6, 7, 8, 9, 10, 11, 12 or 13 different species using a set of conserved, common primers. In some embodiments the set of conserved, common primers includes no more than three primers, for example two or three primers.

Preferably, the set of primers of the kit comprises primers comprising the sequence of SEQ ID NO. 1 or SEQ ID NO. 2 and a primer comprising the sequence of SEQ ID NO. 3. In some embodiments, the set of primers consists of three primers comprising the sequences of SEQ ID NO. 1, SEQ ID NO. 2 and SEQ ID NO. 3 respectively. Preferably, the primers consist of the sequences of SEQ ID NO. 1, SEQ ID NO. 2 and SEQ ID NO. 3. Any one or more of the primers may be adapted by the addition of an adaptor, tag or universal sequence. In some embodiments of the kits, the primer of SEQ ID NO.1 may be replaced by a primer comprising or consisting of the sequence of SEQ ID NO. 4, the primer of SEQ ID NO. 2 may be replaced by a primer comprising or consisting of the sequence of SEQ ID NO. 5 and/or the primer of SEQ ID NO. 3 may be replaced by one or more primers comprising or consisting of the sequences of SEQ ID NO.s 6 to 13.

The invention further provides a kit for quantitative PCR analysis of a species specific target sequence in a sample, said kit comprising a set of primers and probes for the amplification and real-time detection of a sequence from the myostatin gene, said sequence being selected to allow for species specific amplification and/or detection. In some embodiments, the species specific amplification and/or detection can distinguish between species from a genus selected from *Sus, Gallus, Bos, Ovis, Equus, Meleagris, Felis, Capra, Antilocapra, Cervus, Anas, Anser* and *Coturnix,* for example, *Sus scrofa, Gallus gallus, Bos Taur us, Ovis aries, Equus caballus, Meleagris gallopavo, Felis catus, Capra hircus, Antilocapra Americana, Cervus elaphus, Anas platyrhyncho, Anser anser* and *Coturnix chinensis.*

In preferred embodiments, the set of primers and probes is selected from at least one of:
a) primers comprising or consisting of the sequences of SEQ ID NOS. 17 and 18 and a probe comprising or consisting of the sequence of SEQ ID NO. 19 for the quantitation of DNA sequences from pork (*Sus scrofa*); or
b) primers comprising or consisting of the sequences of SEQ ID NOS. 20 and 16 and a probe comprising or consisting of the sequence of SEQ ID NO. 21 for the quantitation of DNA sequences from chicken (*Gallus gallus*); or
c) primers comprising or consisting of the sequences of SEQ ID NOS. 22 and 16 and a probe comprising or consisting of the sequence of SEQ ID NO. 23 for the quantitation of DNA sequences from beef (*Bos taurus*); or
d) primers comprising or consisting of the sequences of SEQ ID NOS. 24 and 16 and a probe comprising or consisting of the sequence of SEQ ID NO. 25 for the quantitation of DNA sequences from sheep (*Ovis aries*); or
e) primers comprising or consisting of the sequences of SEQ ID NOS. 26 and 27 and a probe comprising or consisting of the sequence of SEQ ID NO. 28 for the quantitation of DNA sequences from horse (*Equus caballus*); or
f) primers comprising or consisting of the sequences of SEQ ID NOS. 29 and 16 and a probe comprising or consisting of the sequence of SEQ ID NO. 30 for the quantitation of DNA sequences from turkey (*Meleagris gallopavo*); or
g) primers comprising or consisting of the sequences of SEQ ID NOs. 14 and/or 15 and 16 and a probe comprising or consisting of the sequence of SEQ ID NO. 31 or 32 for the quantitation of DNA from all species in the sample.

In some embodiments of the kit primers having the sequences of SEQ ID NOs 20, 22, 24 and/or 29 may be replaced by primers comprising or consisting of the sequences of SEQ ID NOs 14 and 15.

In some embodiments the kits further contain a probe having a sequence of SEQ ID NO. 31 or SEQ ID NO. 32, which acts as a probe for a control or reference sequence in the MSTN gene.

The invention further provides a kit having some or all of the features recited above for metagenomic analysis and further having some or all of the features recited above for qPCR analysis. Optionally the kit may further comprise any reagent suitable for use in the metagenomic or qPCR steps of the process of the invention.

The invention will now be described in further detail with reference to the following examples and figures, in which:
Figure 1 shows an alignment of myostatin Exon1 from 12 mammalian and bird species (frequently found in meat and meat products).
Figure 2 shows the binding site of primers and probes designed on myostatin exon 1 for pork, beef, horse, sheep, chicken and turkey. (1) represents Forward primer; (3) : reverse primer; (2):Taqman probe
Figure 3 shows an amplification curve and linear regression of real-time PCR detection for beef.
Figure 4 shows metagenomic analysis of meat samples

### Material and methods

### DNA isolation

Twenty-five gram sample of meat or meat product was homogenized for 1 minute in 225 ml in a tempo bag (bioMérieux Basingstoke, England, ref 80015) with sterile physiologic water using a stomacher apparatus. 1.5 ml of the suspension was then used for total genomic DNA extraction using DNeasy Blood & Tissue Kit (QIAGEN, Crawley, UK). The DNA concentrations were evaluated by fluorometric ds DNA quantification using PicoGreen.

### Metagenomic analysis using MSTN gene

### Conserved primers design for pyrosequencing

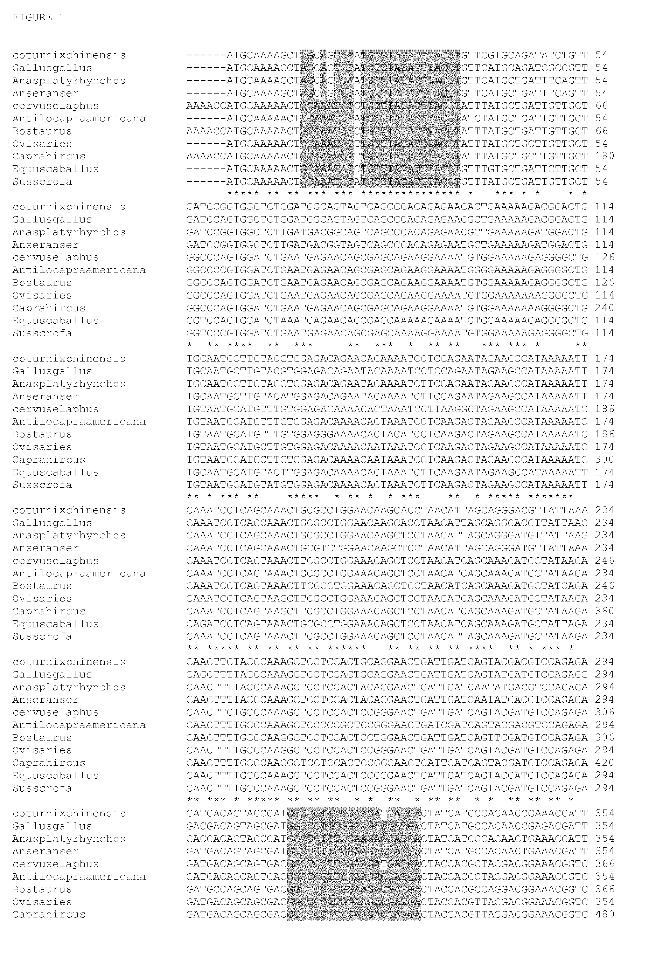

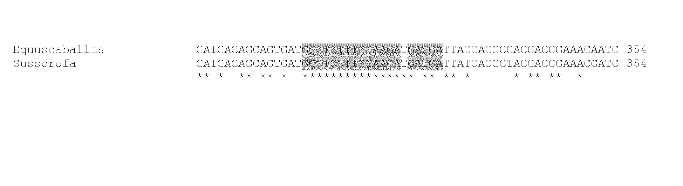

The myostatin exon 1 sequence from 12 frequent species present in meat products (*Sus scrofa, Bos taur us, Gallus gallus, Equus caballus, Ovis aries, Capra hircus, Anas platyrhynchos, Anser anser* and *Coturnix chinensis, Cervus elaphus, Antilocapra Americana*), were aligned to compare conserved area used for the conserved primers design (fig. 1). 3 primers are designed to amplified a PCR fragment of 313 bases (MSTN-E1-UP1 :AGTCTATGTTTATATTTACCT (SEQ ID NO. 1), MSTN-E1-UP2:AATCTYTGTTTATATTTACCT (SEQ ID NO. 2), MSTN-E1-DN :TCATCRTCTTCCAARGAGCC (SEQ ID NO. 3)). The amplified fragment shows a sequence diversity sufficient to identify species by sequencing. The oligonucleotide design included 454 Life Sciences A or B sequencing titanium adapters (Roche Diagnostics, Vilvoorde, Belgium) and multiplex identifiers (MIDs) fused to the 5' end of reverse primer.

A full set of primers designed for 454 Life Sciences sequencing is as follows:

Fusion FW : (B adaptator)-(MSTN-E1-UP)

MSTNE1-UP1:5'-CCTATCCCCTGTGTGCCTTGGCAGTCTCAGAGTCTATGTTTATATTTACCT-3' (SEQ ID NO. 4)
MSTNE1-UP2:5'-CCTATCCCCTGTGTGCCTTGGCAGTCTCAGAATCTYTGTTTATATTTACCT-3' (SEQ ID NO. 5)

Fusion Rev : (A adaptator)-Key-MID-( MSTN-E1-DN )

MSTNE1-DN-MID100 :5'-CCATCTCATCCCTGCGTGTCTCCGACTCAGAGACTGCACTCATCRTCTTCCAARGAGCC-3 (SEQ ID
NO. 6)
MSTNE1-DN-MID101 :5'-CCATCTCATCCCTGCGTGTCTCCGACTCAGATCGCAGGCTCATCRTCTTCCAARGAGCC-3(SEQ ID
NO. 7)
MSTNE1-DN-UM102 :5'-CCATCTCATCCCTGCGTGTCTCCGACTCAGACGCTCGACATCATCRTCTTCCAARGAGCC-3(SEQ ID
NO. 8)
MSTNE1-DN-UM103 :5'-CCATCTCATCCCTGCGTGTCTCCGACTCAGAGACGCACTCTCATCRTCTTCCAARGAGCC-3(SEQ ID
NO. 9)
MSTNE1-DN-UM104 :5'-CCATCTCATCCCTGCGTGTCTCCGACTCAGAGCACTGTAGTCATCRTCTTCCAARGAGCC-3(SEQ ID
NO. 10)
MSTNE1-DN-UM105 :5'-CCATCTCATCCCTGCGTGTCTCCGACTCAGATCAGACACGTCATCRTCTTCCAARGAGCC-3(SEQ ID
NO. 11)
MSTNE1-DN-UM106 :5'-CCATCTCATCCCTGCGTGTCTCCGACTCAGATATCGCGAGTCATCRTCTTCCAARGAGCC-3(SEQ ID
NO. 12)
MSTNE1-DN-UM107 :5'-CCATCTCATCCCTGCGTGTCTCCGACTCAGCGTGTCTCTATCATCRTCTTCCAARGAGCC-3(SEQ ID
NO. 13)

### MSTN gene library construction and pyrosequencing

The amplification mix contained 5 U of FastStart high fidelity polymerase (Roche Diagnostics, Vilvoorde, Belgium), 1x enzyme reaction buffer, 200 µM dNTPs (Eurogentec, Liège, Belgium), 0.2 µM of each primer and 100 ng of genomic DNA in a volume of 25 µl. Thermocycling conditions consisted of a denaturation at 94°C for 10 min followed by 25 cycles of 94°C for 30 s, 50°C for 30 s, 72°C for 1 min and a final elongation step of 7 min at 72°C. These amplifications were performed on an Ep Master system gradient apparatus (Eppendorf, Hamburg, Germany). The PCR products of approximately 313 bp were checked by gel electrophoresis and purified using the AMPure kit (Agencourt Bioscience Corporation, Beverly, USA) to remove amplicons shorter than 100 bp. Equal amounts of each of the PCR products were pooled and subsequently amplified by emulsion PCR before sequencing. Pyrosequencing was performed with the Roche 454 GS Junior Sequencer (Roche, Basel, Switzerland).

### Bioinformatic analysis

Image and data processing for amplicon sequencing was performed using the Genome Sequencer FLX System Software Package 2.3 (Roche, Basel, Switzerland). The Mothur program was used for the first part of the sequences analysis (SCHLOSS et al., 2009). The shorter sequences were discarded. The sequences with ambiguous bases or homopolymers longer than ten nucleotides and with an average quality score lower than 25 were removed along with tag and primer sequences. Sequences from multiplexed samples were assigned based on the presence of the unique barcodes assigned to each sample. A distance matrix was prepared, (distance= 0.01) and the sequences were clustered to operational taxonomic units (OTUs). The representative sequences of each OTU were compared to the NCBI database using the Basic Local Alignment Search Tool (BLASTN).

### Quantification by qPCR using MSTN gene

### Oligonucleotides primers and probes

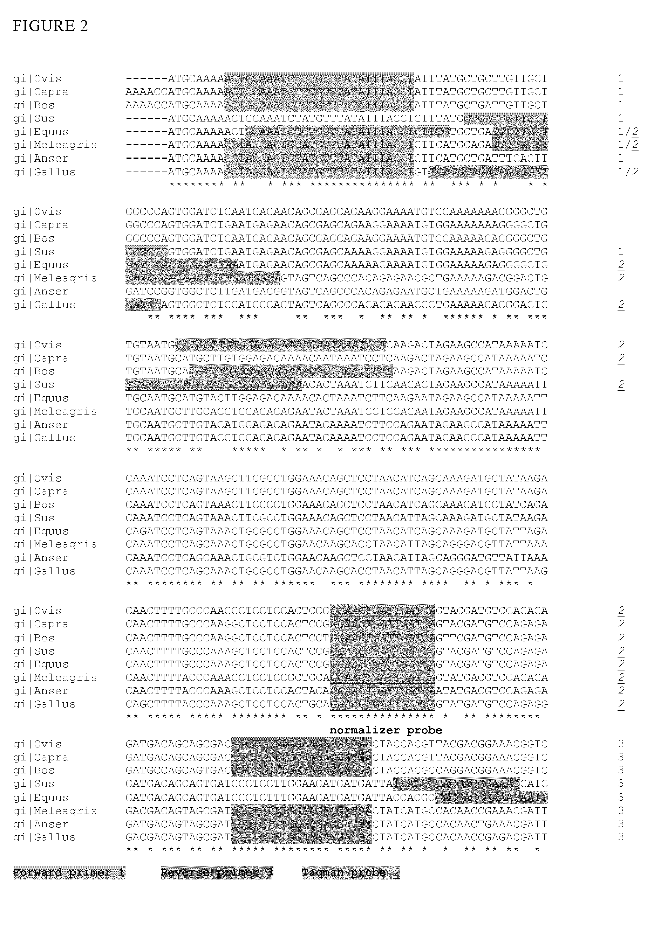

A TaqMan-based real-time Polymerase Chain Reaction (qPCR) assay was developed for quantify 6 frequent species present in meat products (*Sus scrofa, Bos taurus, Gallus gallus, Equus caballus, Ovis aries* and *Meleagris gallopavo*). By aligning the myostatin Exon 1 DNA sequence of these species according to the NCBI database forward and reverse primers and Taqman probes were designed for each meat-specific region (fig.2). The list of these primers and probes is shown in Table 1.

**Table 1 : Sequences of primers and probes used in this study.**

| | | **sequence** |
|---|---|---|
| | forward primer | 5'-CTGATTGTTGCTGGTCCC-3' |
| **pork** | reverse primer | 5'-GTTTCCGTCGTAGCGTGA-3' |
| | Taqman probe | 5'-(FAM)-TGTAATGCATGTATGTGGAGACAAA-(BHQ)-3' |
| | forward primer | 5'-GCTAGCAGCTATGTTTATATTTACCT-3' |
| **chicken** | reverse primer | 5'-TCATCRTCTTCCAARGAGCC-3' |
| | Taqman probe | 5'-(FAM)-TCATGCAGATCGCKGTTGATCC-(BHQ)-3' |
| | forward primer | 5'-ACTGCAAATCTCTGTTTACCT-3' |
| **beef** | reverse primer | 5'-TCATCRTCTTCCAARGAGCC-3' |
| | Taqman probe | 5'-(FAM)-TGTTTGTGGAGGGAAAACACTACATCCTC-(BHQ)-3' |
| | forward primer | 5'-ACTGCCAAATCTTTGTTTATATTTACCT-3' |
| **sheep** | reverse primer | 5'-TCATCRTCTTCCAARGAGCC-3' |
| | Taqman probe | 5'-(FAM)-CATGCTTGTGACAAAACAATAAATCCT-(BHQ)-3' |
| | forward primer | 5'-GCAAATCTCTGTTTATATTTACCTGTTTG-3' |
| **horse** | reverse primer | 5'-GGTAATGATTGTTTCCGTCGTC-3' |
| | Taqman probe | 5'-(FAM)-TTCTTGCTGGTCCAGTGGATCTAA-[BHQ]-3' |
| | forward primer | 5'-GCTAGCAGTCTATGTTTATATTTACCT-3' |
| **turckey** | reverse primer | 5'-TCATCRTCTTCCAARGAGCC-3' |
| | Taqman probe | 5'-(FAM)-TGCAGATTTTAGTTCACGGT-(BHQ)-3' |
| | forward primer | 5'-GCTAGCAGTCTATGTTTATATTTACCT-3' |
| **normalizer control** | reverse primer | 5'-TCATCRTCTTCCAARGAGCC-3' |
| | Taqman probe | 5'-(FAM)-GGAACTGATTGATCA-(BHQ)-LNA-3' |

The sequences in Table 1 have the following SEQ ID NOs:
Pork forward primer: SEQ ID NO. 17. Pork reverse primer: SEQ ID NO.18. Pork Taqman probe: SEQ ID NO. 19.
Chicken forward primer: SEQ ID NO. 20. Chicken reverse primer: SEQ ID NO.16. Chicken Taqman probe: SEQ ID NO. 21.
Beef forward primer: SEQ ID NO. 22. Beef reverse primer: SEQ ID NO.16. Beef Taqman probe: SEQ ID NO. 23.
Sheep forward primer: SEQ ID NO. 24. Sheep reverse primer: SEQ ID NO. 16. Sheep Taqman probe: SEQ ID NO. 25.
Horse forward primer: SEQ ID NO. 26. Horse reverse primer: SEQ ID NO.27. Horse Taqman probe: SEQ ID NO. 28.
Turkey forward primer: SEQ ID NO. 29. Turkey reverse primer: SEQ ID NO.30. Turkey Taqman probe: SEQ ID NO. 31.
Normalizer forward primer: SEQ ID NO. 14. Normalizer reverse primer: SEQ ID NO. 16. Normalizer Taqman probe: SEQ ID NO. 31. A further normalizer Taman probe that may be use is:
   FAM-AGTGGAGGAGCYTTGGGYAAAAG-BHQ (SEQ ID NO. 32)

The forward primers for chicken, beef, sheep and turkey noted above may be replaced by a mixture of the following two primers:
GCTAGCAGTCTATGTTTATATTTACCT (SEQ ID NO. 14)
ACTGCAAATCTYTGTTTATATTTACCT (SEQ ID NO. 15).

### Conventional PCR protocol

Real-time PCR was performed using the LightCycler 480 system (Roche, Basel, Switzerland). The PCR reaction mixtures were placed in a 12 µl final volume containing 6 µl of LC480 probe master mix (Roche, Basel, Switzerland), 2 µl of template DNA (at 5ng/µl), 0,25 µl of primer pair (10µM each), 0,125 µl of Taqman probe (10µM). The reaction conditions included the initiation step for 10 min at 95°C, followed by 40 cycles of 15s at 95°C and 1 min at 60°C. The real-time system is supplied with the Lightcycler 480 Software version 1.5 using unique Roche algorithms for highly accurate and robust automated data analysis

### Specificity and sensitivity test.

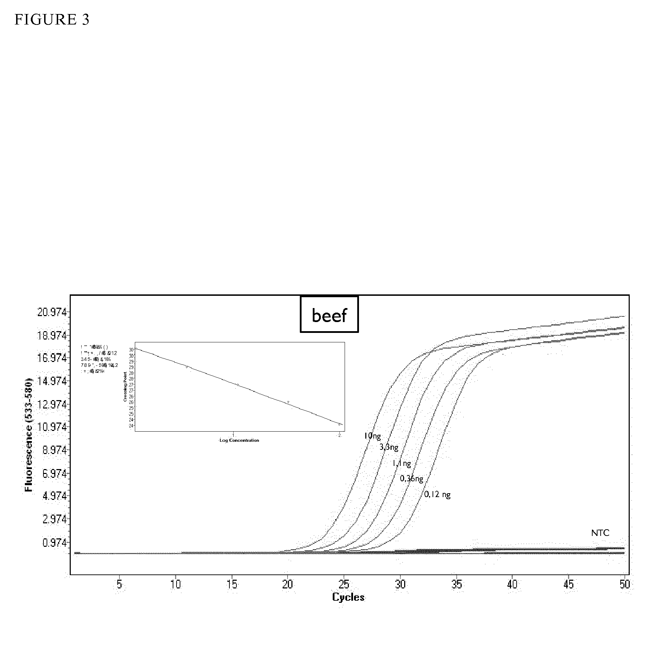

The specificity of each species test was confirmed by the amplification of 100ng of chicken, turkey, horse, donkey, pork, bovine and ovine genomic DNA and a negative control without DNA (NTC). Each assay was tested against DNA from all six species, against its target species and the 5 remaining species to confirm specificity. In sensitivity test of the specific primers and probes, PCR amplifications were examined between specific primers and 3-fold serial dilutions of DNAs isolated from the meat of each target species ranging from 10ng to 0,4 ng. The standard curves for all the 6 detection systems were constructed by using the Cp value obtained from the corresponding DNA concentration (fig 3).

### Results

In this study, metagenomic and real-time PCR assays based on the amplification of same fragment of genomic myostatin gene were developed and evaluated for detection and quantification of 6 frequent species present in meat and meat products (pork, beef, horse, sheep, chicken and turkey).

### Metagenomic analysis using MSTNgene

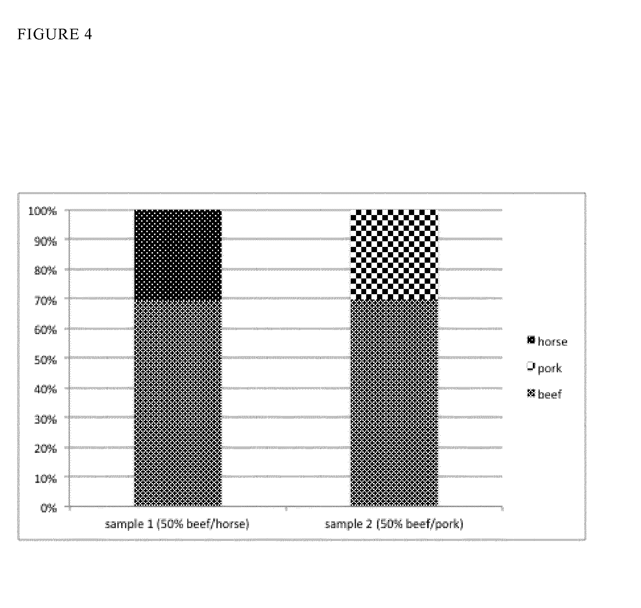

The metagenomic assay was applied to two commercial meat preparations, the first composed of 50% of beef and 50% of pork meat, the second composed of 50% of beef and 50% of horse meat. The treated sequences were compared to the NCBI database using the Basic Local Alignment Search Tool (fig. 4). The metagenomic analysis allowed identification of all the meat components but the proportions are not fully representative. The proportion of beef are more important in the two samples tested to the detriment of the 2 others species. To obtain more quantitative analysis its important to avoid competition between meat components thus to analyse in the early part of the exponential phase because of all reagents are still in excess and the species which is present in a low amount will not compete with the others.

### Quantification by qPCR using MSTN gene

Species specific primers and Taqman probes were designed and amplification conditions were optimized by using these primers and probes.

### Specificity of the Taqman probe systems.

The specificity of the 6 systems were tested for 8 common and commercial meat 100% pure species (pork, beef, horse, sheep, chicken, turkey, rabbit, duck). No cross-reaction was obtained with any of the non target species DNA (table 2).

**Table 2 : Specificity and sensitivity test results obtained with the species specific primers-probe sets. ND = not detected.**

| | | **Average Cp value ±SD** | | | | | |
|---|---|---|---|---|---|---|---|
| **species** | **DNA concentration (ng)** | **pork** | **beef** | **sheep** | **horse** | **turkey** | **chicken** |
| target species | 10 | 23,57 ± 0,11 | 23,99 ± 0,39 | 23,5 ± 0,05 | 24,14 ± 0,43 | 22,69 ± 0,15 | 23,34 ± 0,48 |
| | 3,3 | 25,16 ± 0,06 | 25,66 ± 0,19 | 25,06 ± 0,08 | 24,59 ± 0,62 | 24,29 ± 0,22 | 24,49 ± 0,51 |
| | 1,1 | 26,86 ± 0,02 | 27,28 ± 0,4 | 26,78 ± 0,05 | 26,99 ± 0,15 | 25,96 ± 0,15 | 26,08 ± 0,47 |
| | 0,36 | 28,47 ± 0,12 | 28,8 ± 0,25 | 28,45 ± 0,23 | 28,99 ± 0,64 | 28,07 ± 0,74 | 27,98 ± 0,19 |
| | 0,12 | 30,08 ± 0,03 | 30,44 ± 0,32 | 29,93 ± 0,11 | 30,0 ± 0,31 | 29,29 ± 0,11 | 29,73 ± 0,06 |
| | 0,04 | ± | 31,8 ± 0,14 | 31,4 ± 0,58 | 32,12 ± 0,61 | 30,94 ± 0,16 | 31,14 ± 0,2 |
| qPCR efficiency | | 1,937 | 1,968 | 1,886 | 1,991 | 1,963 | 1,99 |
| chicken | 10 | ND | ND | ND | ND | ND | 23,231±0,4 |
| turkey | 10 | ND | ND | ND | ND | 22,72 ±0,13 | ND |
| horse | 10 | ND | ND | ND | 24,04 ±0,37 | ND | ND |
| donkey | 10 | ND | ND | ND | ND | ND | ND |
| pork | 10 | 23,39 ± 0,41 | ND | ND | ND | ND | ND |
| beef | 10 | ND | 24,01 ± 0,32 | ND | ND | ND | ND |
| sheep | 10 | ND | ND | 23,46 ± 0,082 | ND | ND | ND |
| rabbit | 10 | ND | ND | ND | ND | ND | ND |
| duck | 10 | ND | ND | ND | ND | ND | ND |

### Sensitivity and linearity of Taqman probe systems.

The sensitivity and linearity of the 6 Taqman systems were tested by using a 3-fold dilutions series of the genomic DNA obtained from each target species starting with 10ng target DNA. The standard curve was generated by plotting the resulting Cp value compared with the logarithm of the target DNA concentration to test linearity. These linear regression were made by the LC480 software using the own Roche algorithms. The efficiency values (provided by the Roche algorithms) for the pork; beef; sheep ; horse ; turkey and chicken systems were respectively 1,937 ; 1,968 ; 1,886 ; 1,991 ; 1,963 and 1,99. As is well known, an acceptable range to determine the efficiency of a qPCR reaction and thus the linear relationship between the Cp value and the DNA concentration is between 1,8 and 2,2 (Efficiency= -1+10^{(-1/slope);} a perfect efficiency= 2).

### Normalization methods

Sources of variability could be diversified (the nature and amount of starting sample, the DNA isolation process or lastly real-time PCR amplification). Normalization is essentially the act of neutralizing the effects of variability which could prevent the ability to compare data and lead to erroneous conclusions. The use of a normalizer gene is the most thorough method of addressing almost every source of variability in real-time PCR. In this study we have decided to use as normalizer a highly conserved region from myostatin fragment amplified (fig.2). This normalizer should have a similar amplification efficiency because we use the similar primers to amplify it.

To quantify data we could use comparative quantification and the ΔΔCₜ method. With this method Cₜs for the gene of interest in both the test sample and calibrator sample (here we use a 100% pure target species) are adjusted in relation to the normalizer gene Cₜ from the same 2 samples (ΔC_{t sample}-ΔC_{t calibrator} = ΔΔCₜ). The resulting ΔΔCₜ value is incorporated to determine the fold difference in expression (fold difference= 2-^{ΔΔC}ₜ).

The invention is not limited by the Examples and the skilled person will appreciate alternatives and modifications that might be made to any step or component as herein described. The invention is therefore limited only by the scope of the claims.

## Claims

1. A method for determining the species origin(s) of DNA in a sample comprising conducting metagenomic sequence analysis of the sample to identify DNA from at least one species, followed by species specific quantitative polymerase chain reaction (qPCR) to amplify and quantify a species specific target from the identified species DNA.

2. A method of claim 1 wherein the qPCR step is selected from qPCR using an intercalating dye and qPCR using a labelled reporter primer or probe.

3. A method of claim 2 wherein the labelled reporter primer or probe is part of a fluorescence/quencher reporter system or other fluorescence reporter system in which amplification of the species specific DNA target results in a reduction or elimination of fluorescence quenching, or other increase in fluorescence, to result in a detectable fluorescent signal.

4. A method of claim 3 wherein the reporter system comprises a hydrolysis probe, a molecular beacon probe, a pair of dual hybridization probes, an amplifluor primer system, a scorpion primer system, a light-upon-extension system or a QZyme primer system.

5. A method of claim 2 wherein the intercalating dye is selected from SYBRGreen and EvaGreen.

6. A method of any preceding claim wherein the sample is a food sample, e.g. a meat sample.

7. A method of any preceding claim wherein the metagenomic analysis comprises amplification and sequencing of a metagenomic target DNA sequence, said metagenomic target DNA sequence being selected to allow for: i) co-amplification of the metagenomic target DNA sequence from a plurality of species using a set of conserved, common primers; and ii) the identification of species origin of any amplified metagenomic target DNA on the basis of its sequence

8. A method of claim 7 wherein the metagenomic target sequence has the same number of copies per genome in each of the species suspected as being present in the sample, so as to further allow for a determination of the relative proportions of the different identified DNAs.

9. A method of claim 7 or claim 8 wherein the metagenomic target DNA sequence is a part of the myostatin (MSTN) gene.

10. A method of any preceding claim wherein the species specific target DNA sequence is part of the MSTN gene.

11. A method of claim 9 or claim 10 wherein the metagenomic target DNA sequence is amplified by a set of conserved primers comprising at least a primer comprising or consisting of the sequence of SEQ ID NO. 1 or SEQ ID NO. 2, and a primer comprising or consisting of the sequence of SEQ ID NO. 3.

12. A method of claim 11 wherein the metagenomic target DNA sequence is amplified by a set of conserved primers comprising a primer comprising or consisting of the sequence of SEQ ID NO. 1 and a primer comprising or consisting of the sequence of SEQ ID NO. 2 and a primer comprising or consisting of the sequence of SEQ ID NO. 3.

13. A method of any of claims 10 to 12 wherein at least one of the primers is modified by the addition of an adaptor sequence, a tag or a universal sequence, optionally wherein the set of primers comprises or consists of primers having the sequences of SEQ ID NOs. 4 to 13.

14. A method of any preceding claim wherein the metagenomic target DNA sequence is sequenced by a process for massively parallel sequencing.

15. A method of claim 14 wherein the process for massively parallel sequencing is selected from emulsion sequencing, and solid phase sequencing.

16. A method of any preceding claim wherein the species specific target DNA sequence is amplified by a set of primers and the resulting amplicon is detected by a labelled hybridisation probe, the primers and probes being selected from the following:
a) primers comprising or consisting of the sequences of SEQ ID NOS. 17 and 18 and a probe comprising or consisting of the sequence of SEQ ID NO. 19 for the quantitation of DNA sequences from pork (*Sus scrofa*); or
b) primers comprising or consisting of the sequences of SEQ ID NOS. 20 and 16 and a probe comprising or consisting of the sequence of SEQ ID NO. 21 for the quantitation of DNA sequences from chicken (*Gallus gallus*); or
c) primers comprising or consisting of the sequences of SEQ ID NOS. 22 and 16 and a probe comprising or consisting of the sequence of SEQ ID NO. 23 for the quantitation of DNA sequences from beef (*Bos taurus*); or
d) primers comprising or consisting of the sequences of SEQ ID NOS. 24 and 16 and a probe comprising or consisting of the sequence of SEQ ID NO. 25 for the quantitation of DNA sequences from sheep (*Ovis aries*); or
e) primers comprising or consisting of the sequences of SEQ ID NOS. 26 and 27 and a probe comprising or consisting of the sequence of SEQ ID NO. 28 for the quantitation of DNA sequences from horse (*Equus caballus*); or
f) primers comprising or consisting of the sequences of SEQ ID NOS. 29 and 16 and a probe comprising or consisting of the sequence of SEQ ID NO. 30 for the quantitation of DNA sequences from turkey (*Meleagris gallopavo*); or
g) primers comprising or consisting of the sequences of SEQ ID NOs. 14 and/or 15 and 16 and a probe comprising or consisting of the sequence of SEQ ID NO. 31 or 32 for the quantitation of DNA from all species in the sample.

17. A method of claim 16 wherein any one or more of primers having of SEQ ID NOs. 20, 22, 24 or 29 are replaced by a mixture of primers comprising or consisting of the sequences of SEQ ID NOs. 14 and 15.

18. The use of the primers as defined in claims 11 to 13 together with the primers and probes of claim 16 or 17 for the sequential metagenomic identification and species specific quantitation of DNA in a sample, the selection of species specific primers and probes employed for quantitation being chosen on the basis of the metagenomic identification of species DNA present in the sample.

19. A use of claim 18 wherein the sample is a food sample, such as a meat sample.

20. A method of any of claims 1 to 15 for the identification and quantification of at least one species specific target sequence in a sample from any one or more of a species from a genus selected from *Sus, Gallus, Bos, Ovis, Equus, Meleagris, Felis, Capra, Antilocapra, Cervus, Anas, Anser* and *Coturnix,* for example, *Sus scrofa, Gallus gallus, Bos Taur us, Ovis aries, Equus caballus, Meleagris gallopavo, Felis catus, Capra hircus, Antilocapra Americana, Cervus elaphus, Anas platyrhyncho, Anser anser* and *Coturnix chinensis.*

21. A kit for metagenomic identification of DNA present in a sample, comprising a set of primers for the metagenomic amplification of a metagenomic target sequence in the myostatin gene, said metagenomic target sequence being selected to allow for: i) co-amplification of the metagenomic target DNA sequence from a plurality of species using a set of conserved, common primers; and ii) the identification of species origin of any amplified metagenomic target DNA on the basis of its sequence.

22. A kit of claim 21 wherein the metagenomic target sequence has the same number of copies per genome in each of the species suspected as being present in the sample, so as to further allow for a determination of the relative proportions of the different identified DNAs.

23. A kit of claim 21 or claim 22 wherein the metagenomic target sequence is selected to allow for amplification of DNA from at least 2, 3, 4, 5, 6, 7, 8, 9, 10, 11, 12 or 13 different species using a set of conserved, common primers.

24. A kit of any of claims 21 to 23 wherein the set of conserved, common primers includes no more than three primers, for example two or three primers.

25. A kit of any of claims 21 to 24 wherein the set of primers of the kit comprises primers comprising or consisting of the sequence of SEQ ID NO. 1 or SEQ ID NO. 2 and a primer comprising or consisting of the sequence of SEQ ID NO. 3.

26. A kit of claim 25 wherein the set of primers consists of three primers comprising or consisting of the sequences of SEQ ID NO. 1, SEQ ID NO. 2 and SEQ ID NO. 3 respectively.

27. A kit of claim 24 to 26 wherein at least one of the primers is modified by the addition of an adaptor sequence, tag or universal sequence, optionally wherein the set of primers comprises or consists of primers having the sequences of SEQ ID NOs. 4 to 13.

28. A kit for quantitative PCR analysis of a species specific target sequence in a sample, said kit comprising a set of primers and probes for the amplification and real-time detection of a sequence from the myostatin gene, said sequence being selected to allow for species specific amplification and/or detection.

29. A kit of claim 28 wherein the species specific amplification and/or detection can distinguish between species from a genus selected from *Sus, Gallus, Bos, Ovis, Equus, Meleagris, Felis, Capra, Antilocapra, Cervus, Ana, Anser* and *Coturnix,* for example, *Sus scrofa, Gallus gallus, Bos Taur us, Ovis aries, Equus caballus, Meleagris gallopavo, Felis catus, Capra hircus, Antilocapra Americana, Cervus elaphus, Anas platyrhyncho, Anser anser* and *Coturnix chinensis.*

30. A kit of claim 28 wherein the set of primers and probes is selected from at least one of:
a) primers comprising or consisting of the sequences of SEQ ID NOS. 17 and 18 and a probe comprising or consisting of the sequence of SEQ ID NO. 19 for the quantitation of DNA sequences from pork (*Sus scrofa*); or
b) primers comprising or consisting of the sequences of SEQ ID NOS. 20 and 16 and a probe comprising or consisting of the sequence of SEQ ID NO. 21 for the quantitation of DNA sequences from chicken (*Gallus gallus*); or
c) primers comprising or consisting of the sequences of SEQ ID NOS. 22 and 16 and a probe comprising or consisting of the sequence of SEQ ID NO. 23 for the quantitation of DNA sequences from beef (*Bos taur us*); or
d) primers comprising or consisting of the sequences of SEQ ID NOS. 24 and 16 and a probe comprising or consisting of the sequence of SEQ ID NO. 25 for the quantitation of DNA sequences from sheep (*Ovis aries*); or
e) primers comprising or consisting of the sequences of SEQ ID NOS. 26 and 27 and a probe comprising or consisting of the sequence of SEQ ID NO. 28 for the quantitation of DNA sequences from horse (*Equus caballus*); or
f) primers comprising or consisting of the sequences of SEQ ID NOS. 29 and 16 and a probe comprising or consisting of the sequence of SEQ ID NO. 30 for the quantitation of DNA sequences from turkey (*Meleagris gallopavo*); or
g) primers comprising or consisting of the sequences of SEQ ID NOs. 14 and/or 15 and 16 and a probe comprising or consisting of the sequence of SEQ ID NO. 31 or 32 for the quantitation of DNA from all species in the sample.

31. A kit of claim 30 wherein any one or more of primers having of SEQ ID NOs. 20, 22, 24 or 29 are replaced by a mixture of primers comprising or consisting of the sequences of SEQ ID NOs. 14 and 15.

32. A kit comprising the features of any of claims 21 to 27 further comprising the features of any one of claims 28 to 31.

33. A kit of any of claims 21 to 32 further comprising any reagent suitable for use in the metagenomic or qPCR analysis of the DNA content of a sample.
